# EUROPEAN PATENT APPLICATION

(11) **EP 4 252 766 A1**
(43) Date of publication of application: **04.10.2023**
(21) Application number: 22305435.4
(22) Date of filing: 01.04.2022
(51) Int. Cl.: A61K 38/17, A61P 25/28, A61P 3/08, A61P 3/10, A61P 3/04, A61P 3/06, A61P 19/02, A61P 19/08, A61P 19/10, A61P 17/06, A61P 37/00, A61P 27/02, G01N 33/50

(54) **HIP/PAP PROTEIN OR A DERIVATIVE THEREOF FOR TREATING AND/OR PREVENTING A DISORDER CHARACTERIZED BY A HIGH CXCL5 SERUM LEVEL IN AN INDIVIDUAL**

(71) Applicant: THE HEALTHY AGING COMPANY, 75001 Paris (FR)
(72) Inventor: BRECHOT, Christian, 75006 Paris (FR); AMOUYAL, Gilles, 75016 Paris (FR); AMOUYAL, Paul, 75016 Paris (FR); SANTORO, Lyse, 75016 Paris (FR); ROTH, Fanny, 94800 Villejuif (FR); JAMOT, Laure, 75013 Paris (FR)
(74) Representative: Cabinet Nony

(57) **Abstract**

The present invention relates to the use of the HIP/PAP protein, or a derivative thereof, for treating and/or preventing, in an individual in need thereof, a disorder characterized by a CXCL5 level higher in a biological sample of said individual than in a corresponding biological sample of a healthy individual. It also relates to a method for measuring a response to a HIP/PAP protein, or a derivative thereof, in an individual having a disorder characterized by a CXCL5 level higher in a biological sample of said individual than in a corresponding biological sample of a healthy individual and to a method for determining if a disorder in an individual can be treated by the administration, to the said individual, of a HIP/PAP protein, or a derivative thereof.

## Description

### Field of the invention

The present invention relates to the use of the HIP/PAP protein, or a derivative thereof, in the treatment and prevention of disorders characterized by a CXCL5 level higher in a biological sample of the individual than in a corresponding biological sample of a healthy individual.

The present invention also relates to a method for measuring a response to a HIP/PAP protein, or a derivative thereof, in an individual having a disorder characterized by a CXCL5 level higher in a biological sample of said individual than in a corresponding biological sample of a healthy individual and to a method for determining if a disorder in an individual can be treated by the administration, to the said individual, of a HIP/PAP protein, or a derivative thereof.

### Prior art

CXCL5 (C-X-C motif chemokine 5) is a chemokine belonging to the CXC chemokine family. Inflammation signals such as IL-1 and TNFα activate secretion of CXCL5 from a variety of cells including epithelial cells, keratinocytes, endothelial cells, fibroblasts, neutrophils and monocytes.

Aberrant CXCL5 levels, and in particular overexpression of CXCL5, have been associated with a significant number of disorders, in particular inflammatory and fibrotic diseases. In addition, CXCL5 has also been identified as being implicated in numerous tumors, such as in AihuaLi et al. (Am J Pathol. 2011 Mar; 178(3): 1340-1349) where its overexpression has been described as being associated with poor survival in patients with pancreatic cancer. S.R. Pickens et al. (Angiogenesis. 2011 Dec; 14(4):443-55) described that mice receiving anti-CXCL5 antibodies demonstrated significantly reduced clinical signs of IL-17-arthritis compared to the mice treated with IgG control. Moreover, Xingqing Jia et al. (J Oncol. 2021 May 31;2021:9919494) described that knockdown of CXCL5 inhibits cell proliferation and invasion through the NF-*κ*B signaling pathway in Hepatocellular carcinoma patients. Similarly, Y-L Hsu et al. (Oncogene 32, 4436-4447 (2013)) described that mice treated with anti-CXCL5 antibodies showed decreased metastasis of 4T1 breast cancer cells, inhibition of CXCL5-mediated ERK/Snail signaling being accordingly described as being an attractive therapeutic target for treating metastases in breast cancer patients.

It would accordingly be hilghly interesting to be able to provide a novel agent capable of reducing the concentration of CXCL5 in individuals with a disorder associated with an increased concentration of CXCL5.

Therefore, there is a need for novel actives able to prevent and/or treat disorders associated with an increased concentration of CXCL5, i.e. disorders characterized by a CXCL5 level higher in a biological sample of said individual than in a corresponding biological sample of a healthy individual.

The present invention provides a response to this need.

### Summary of the invention

The present invention relates to a HIP/PAP protein, or a derivative thereof, for its use in treating and/or preventing, in an individual in need thereof, a disorder characterized by a CXCL5 level higher in a biological sample of said individual than in a corresponding biological sample of a healthy individual.

The applicant has demonstrated that, surprisingly, the HIP/PAP protein, or a derivative thereof, is capable of downregulating the production of CXCL5 in an individual with a disorder associated with an increased concentration of CXCL5 and to accordingly improve the condition of said individual.

The HIP/PAP protein may therefore advantageously be used for the treatment of disorders associated with an increased concentration of CXCL5, i.e. disorders characterized by a CXCL5 level higher in a biological sample of the said individual than in a corresponding biological sample of a healthy individual, i.e. in samples from the individual having the disorder and the healthy individual having the same biological nature (liquid sample or tissue sample) and the same biological origin.

The biological sample may in particular be selected from the group consisting of a liquid sample and a tissue sample, more particularly a liquid sample, in particular selected from the group consisting of whole blood sample, plasma sample, serum sample, urine sample and saliva sample, and in particular being a serum sample.

A disorder to be treated and/or prevented according to the invention may be selected from the group consisting of T helper 17 cell-mediated autoimmune disorders; osteoporosis; osteopetrosis; arthritis; retinopathies and Paget's disease.

The T helper 17 cell-mediated autoimmune disorders may be selected from the group consisting of multiple sclerosis, rheumatoid arthritis and psoriasis.

An individual according to the invention may be a mammal, in particular a human being.

A HIP/PAP protein according to the present invention may comprise an amino acid sequence selected from the group consisting of sequences set forth as SEQ ID NO: 1, SEQ ID NO: 2, SEQ ID NO: 3 and SEQ ID NO: 4, in particular may comprise, more particularly may consist in, the sequence set forth as SEQ ID NO: 3.

A HIP/PAP derivative may comprise an amino acid sequence having a sequence identity of at least 80% with the amino acid sequence selected from the group consisting of sequences SEQ ID NO: 1, SEQ ID NO: 2, SEQ ID NO: 3 and SEQ ID NO: 4 and a biological activity of the same nature as the amino acid sequence selected from the group consisting of sequences SEQ ID NO: 1, SEQ ID NO: 2, SEQ ID NO: 3 and SEQ ID NO: 4, and in particular may comprise, and more particularly may consist in, an amino acid sequence having a sequence identity of at least 80% with the amino acid sequence set forth as SEQ ID NO: 3 and a biological activity of the same nature as the amino acid sequence set forth as SEQ ID NO: 3.

The HIP/PAP protein, or a derivative thereof, may be in a composition comprising a physiologically acceptable medium.

The composition may be for oral, sublingual, subcutaneous, intramuscular, intravenous, topical, local, intratracheal, intranasal or rectal administration.

The composition may be for oral, subcutaneous, intravenous, topical or local administration.

The HIP/PAP protein, or the derivative thereof, may be administered to an individual according to the invention in combination with at least one agent known for being useful in the prevention and/or treatment of the disorder of the individual, and in particular in combination with at least one agent known for being useful in the prevention and/or treatment of a T helper 17 cell-mediated autoimmune disorder, in particular selected from the group consisting of multiple sclerosis, rheumatoid arthritis or psoriasis; osteoporosis; osteopetrosis, arthritis; retinopathies and Paget's disease.

The present invention further relates to a method for measuring a response to a HIP/PAP protein, or a derivative thereof, in an individual having a disorder characterized by a CXCL5 level higher in a biological sample of said individual than in a corresponding biological sample of a healthy individual comprising:
(a) determining the CXCL5 level in the biological sample of the individual, in particular in a serum sample;
(b) after step (a), administering to the said individual a HIP/PAP protein, or a derivative thereof;
(c) determining the CXCL5 level, in a biological sample corresponding to the biological sample of step (a), of the individual treated according to step (b) with a HIP/PAP protein, or a derivative thereof; and
(d) comparing the CXCL5 level determined at step (a) with the CXCL5 level determined at step (c).

In a method of the invention, the HIP/PAP protein, or a derivative thereof, may be in a composition comprising a physiologically acceptable medium.

Step (b) of the method according to the invention may last for at least 7 days, in particular at least 2 weeks, more particularly at least 3 weeks.

The present invention also relates to a method for determining if a disorder in an individual can be treated by the administration, to the said individual, of a HIP/PAP protein, or a derivative thereof, comprising:
(a) determining the level of CXCL5 in a biological sample of the individual, in particular in a serum sample; and
(b) comparing the CXCL5 level determined at step (a) with the CXCL5 level of a corresponding biological sample of a healthy individual.

The disorder considered in a method of the present invention may be selected from the group consisting of:
- traumatic brain injury (TBI); foetal, neonate, child or adult traumatic brain injury, in particular caused by a brain hypoxia; cerebellar disease or disorder, such as cerebellar ataxias; Alzheimer's disease; pathological states that precede the occurrence of the Alzheimer's disease *per se,* which includes mind cognitive impairment; stroke, in particular ischemic stroke and hemorrhagic stroke;
- cancers, and in particular lung cancer, including small-cell lung cancer, non-small cell lung cancer, adenocarcinoma of the lung and squamous carcinoma of the lung; cancer of the peritoneum; hepatocellular cancer; gastric or stomach cancer, including gastrointestinal cancer; pancreatic cancer; glioblastoma; cervical cancer; ovarian cancer; liver cancer; bladder cancer; cancer of the urinary tract; breast cancer; colon cancer; rectal cancer; colorectal cancer; small intestine cancer; endometrial or uterine carcinoma; salivary gland carcinoma; kidney or renal cancer; prostate cancer; vulval cancer; thyroid cancer; hepatic carcinoma; anal carcinoma; penile carcinoma; brain tumor; head and neck cancers; melanoma;
- insulin resistance; dyslipidemia, in particular hypercholesterolemia; atherosclerosis, in particular a coronary artery disease, a cerebrovacular disease or lower extremity arterial disease;
- visceral excess weight; obesity; metabolic syndrome; polycystic ovary syndrome; an eating disorder; hepatitis C; hepatic steatosis; sarcopenia; hyperandrogenism; hypercatabolism; undernourishment;
- microbiota-related diseases and disorders, and in particular disease or disorder selected from the group consisting of inflammatory bowel disease (IBD); colitis; gastrointestinal infections; irritable bowel syndrome and other gastrointestinal functional diseases; allergic diseases; cystic fibrosis; atopic dermatitis; neurological diseases, such as autism; anxiety; depression; chronic pain;
- peripheral neuropathies, in particular diabetic peripheral neuropathies; and
- T helper 17 cell-mediated autoimmune disorders, in particular multiple sclerosis; rheumatoid arthritis; psoriasis; osteoporosis; osteopetrosis; arthritis; retinopathies and Paget's disease.

### BRIEF DESCRIPTION OF THE FIGURES

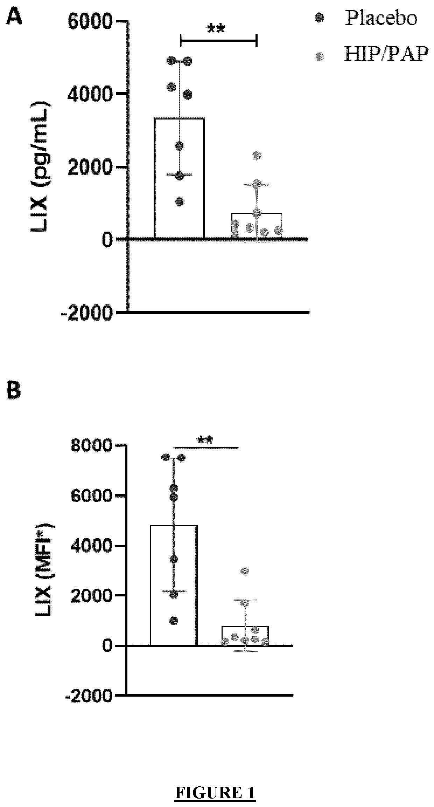

Figure 1, comprised of Figure 1A and Figure 1B, shows LIX/CXCL5 differential expression between HIP/PAP-treated animals and placebo-treated animals of the examples. Figure 1A represents the results from the analysis based on concentrations of LIX/CXCL5 in pg/mL (ordinate) in, from left to right on the abscissa axis, placebo-teated animals or HIP/PAP-treated animals. Figure 1B represents the results from the analysis of the median fluorescence intensity (MFI^{∗}) (ordinate) in, from left to right on the abscissa axis, placebo-teated animals or HIP/PAP-treated animals. Mann-Whitney statisticak test: P<0.0022.

### Detailed description of the invention

### Definitions

In the context of the present invention, the terms "*prevent*", *"prevention"* and "*preventing*" denote the reduction to a lesser degree of the risk or of the probability of occurrence of a given phenomenon, that is to say, in the present invention, the prevention of the onset of a disorder characterized by a CXCL5 level higher in a biological sample of the said individual than in a corresponding biological sample of a healthy individual, and in particular of a disorder selected from the group consisting of T helper 17 cell-mediated autoimmune disorders; osteoporosis; osteopetrosis; arthritis; retinopathies and Paget's disease, more particularly selected from the group consisting of multiple sclerosis; rheumatoid arthritis; psoriasis; osteoporosis; osteopetrosis; arthritis; retinopathies and Paget's disease. The terms "*prevent*", *"prevention"* and "*preventing*" also include preventing the aggravation, reducing the rate of progression or preventing reccurence of the said given phenomen or disorder.

As used herein, the terms "*treating*", "*treatment*" or "*treat*" include alleviation of the symptoms associated with a specific disorder or condition and/or elimination of said symptoms, that is to say, in the present invention, the treatment of a disorder characterized by a CXCL5 level higher in a biological sample of the said individual than in a corresponding biological sample of a healthy individual, and in particular of a disorder selected from the group consisting of T helper 17 cell-mediated autoimmune disorders; osteoporosis; osteopetrosis; arthritis; retinopathies and Paget's disease, more particularly selected from the group consisting of multiple sclerosis; rheumatoid arthritis; psoriasis; osteoporosis; osteopetrosis; arthritis; retinopathies and Paget's disease, even more particularly selected from the group consisting of multiple sclerosis; rheumatoid arthritis; psoriasis; osteoporosis; osteopetrosis; arthritis and Paget's disease and more particularly selected from the group consisting of multiple sclerosis; rheumatoid arthritis; psoriasis; arthritis and Paget's disease.

A *"healthy"* individual refers to the generally accepted definition of this term, i.e. to an individual not suffering from the considered disorder(s).

A *"biological sample"* according to the invention is a liquid sample or a tissue sample, in particular a liquid sample. By definition, a biological sample has been previously isolated from the considered individual.

A *"liquid sample"* according to the invention may be selected from the group consisting of whole blood, plasma, serum, urine and saliva sample, in particular a serum or urine sample, and more particularly a serum sample.

A *"tissue sample"* according to the invention may be selected from the group consisting of a brain tissue, in particular a tissue from the olfactory bulb, amygdala, basal ganglia, thalamus, midbrain, pons, medulla oblongata, hippocampal formation, spinal cord, white matter, cerebral cortex, cerebellum, choroid plexus or hypothalamus; an endocrine tissue, such as a tissue from thyroid gland, parathyroid gland, adrenal grland or pituitary gland; a respiratory system tissue, such as a tissue from the nasopharynx, bronchus or lung; a digestive tract tissue, such as a tissue from oral mucosa, salivary gland, esophagus or tongue; a gastrointestinal tissue, in particular a tissue from the stomach, colon, duodenum, rectum or small intestine; a liver tissue; a pancreas tissue; a kidney tissue; a bladder tissue; a urinary tract tissue; a tissue from testis, epididymis, prostate or seminal vesicle; a tissue from the vagina, uterus, cervix, breast, endometrium, fallopian tube, ovary or placenta tissue; a muscle tissue, such as a tissue from heart muscle, skeletal muscle or smooth muscle; a skin tissue; and a bone marrow or lymphoid tissue, such as a tissue from the bone marrow, the appendix, lymph node, spleen, thymus or tonsil.

A *"corresponding biological sample"* according to the invention means a biological sample having the same biological nature (liquid sample or tissue sample) and the same biological origin as the biological sample referred to. For example, when the biological sample of the individual with a disorder according to the invention is a serum sample, then the corresponding biological sample from the healthy individual is also a serum sample.

The term "*physiologically acceptable medium"* is intended to denote a medium which is compatible with the body of the individual to whom said composition must be administered. It is, for example, a non-toxic solvent such as water. In particular, said medium is compatible with oral, sublingual, subcutaneous, intramuscular, intravenous, topical, local, intratracheal, intranasal or rectal administration, and more particularly with oral, subcutaneous, intravenous, topical or local administration.

As used herein, the *"percentage of identity"* between two amino acid sequences is determined by comparing both optimally aligned sequences through a comparison window.

The portion of the amino acid sequence in the comparison window may thus include additions or deletions (for example "gaps") as compared to the reference sequence (which does not include these additions or these deletions) so as to obtain an optimal alignment between both sequences.

The terms *"sequence homology"* or *"sequence identity"* or *"homology"* or *"identity"* are used interchangeably herein. For the purpose of the invention, it is defined here that in order to determine the percentage of sequence homology or sequence identity of two amino acid sequences, the sequences are aligned for optimal comparison purposes. In order to optimize the alignment between the two sequences gaps may be introduced in any of the two sequences that are compared. Such alignment can be carried out over the full length of the sequences being compared. Alternatively, the alignment may be carried out over a shorter length, for example over about 20, about 50, about 100 or more nucleic acids/based or amino acids. The sequence identity is the percentage of identical matches between the two sequences over the reported aligned region.

A comparison of sequences and determination of percentage of sequence identity between two sequences can be accomplished using a mathematical algorithm. The skilled person will be aware of the fact that several different computer programs are available to align two sequences and determine the identity between two sequences (Kruskal, J. B. (1983) An overview of sequence comparison In D. Sankoff and J. B. Kruskal, (ed.), Time warps, string edits and macromolecules: the theory and practice of sequence comparison, pp. 1-44 Addison Wesley).

The percent sequence identity between two amino acid sequences may be determined using the Needleman and Wunsch algorithm for the alignment of two sequences. (Needleman, S. B. and Wunsch, C. D. (1970) J. Mol. Biol. 48, 443-453). Both amino acid sequences can be aligned by the algorithm. The Needleman-Wunsch algorithm has been implemented in the computer program NEEDLE.

For the purpose of the invention, the NEEDLE program from the EMBOSS package was used (version 2.8.0 or higher, EMBOSS: The European Molecular Biology Open Software Suite (2000) Rice, P. LongdenJ. and Bleasby,A. Trends in Genetics 16, (6) pp276- 277, http://emboss.bioinformatics.nl/). For protein sequences, EBLOSUM62 is used for the substitution matrix. The optional parameters used are a gap opening penalty of 10 and a gap extension penalty of 0.5. No end gap penalty is added. In the Output section, Yes has been indicated in response to the question "Brief identity and similarity" and "SRS pairwise" indicated as Output alignment format.

After alignment by the program NEEDLE as described above the percentage of sequence identity between a query sequence and a sequence of the invention is calculated as follows: Number of corresponding positions in the alignment showing an identical amino acid in both sequences divided by the total length of the alignment after subtraction of the total number of gaps in the alignment. The identity defined as herein can be obtained from NEEDLE by using the NOBRIEF option and is labeled in the output of the program as "longest-identity".

The similarity of amino acid sequences, i.e. the percentage of sequence identity, can be determined via sequence alignments using several other art-known algorithms, preferably with the mathematical algorithm of Karlin and Altschul (Karlin & Altschul (1993) Proc. Natl. Acad. Sci. USA 90: 5873-5877), with hmmalign (HMMER package, http://hmmer.wustl.edu/) or with the CLUSTAL algorithm (Thompson, J. D., Higgins, D. G. & Gibson, T. J. (1994) Nucleic Acids Res. 22, 4673-80) available e.g. on https://www.ebi.ac.uk/Tools/msa/clustalo/ or the GAP program (mathematical algorithm of the University of Iowa) or the mathematical algorithm of Myers and Miller (1989 - Cabios 4: 11-17) or Clone Manager 9. Preferred parameters used are the default parameters as they are set on https://www.ebi.ac.uk/Tools/msa/clustalo/.

The grade of sequence identity (sequence matching) may be calculated using e.g. BLAST, BLAT or BlastZ (or BlastX). A similar algorithm is incorporated into the BLASTN and BLASTP programs of Altschul et al (1990) J. Mol. Biol. 215, 403-410. BLAST polynucleotide searches are performed with the BLASTN program, score = 100, word length = 12, to obtain polynucleotide sequences that are homologous to those nucleic acids which encode the relevant protein.

BLAST protein searches are performed with the BLASTP program, score = 50, word length = 3, to obtain amino acid sequences homologous to the SHC polypeptide. To obtain gapped alignments for comparative purposes, Gapped BLAST is utilized as described in Altschul et al (1997) Nucleic Acids Res. 25, 3389-3402. When utilizing BLAST and Gapped BLAST programs, the default parameters of the respective programs are used. Sequence matching analysis may be supplemented by established homology mapping techniques like Shuffle-LAGAN (Brudno M., Bioinformatics 2003b, 19 Suppl 1: 154-162) or Markov random fields. When percentages of sequence identity are referred to in the present application, these percentages are calculated in relation to the full length of the longer sequence, if not specifically indicated otherwise.

In particular embodiments, % identity between two sequences is determined using CLUSTAL O (version 1.2.4).

As used herein, the term *"polypeptide"* refers to a molecule comprising amino acid residues linked by peptide bonds and containing more than five amino acid residues. The amino acids are identified by either the single-letter or three-letter designations. The term *"protein"* as used herein is synonymous with the term *"polypeptide"* and may also refer to two or more polypeptides. Thus, the terms *"protein", "peptide"* and *"polypeptide"* can be used interchangeably. Polypeptides may optionally be modified (e.g., glycosylated, phosphorylated, acylated, farnesylated, prenylated, sulfonated, and the like) to add functionality.

### HIP/PAP protein and derivative thereof according to the invention

The HIP/PAP protein is known for its anti-apoptotic and mitogenic activity on hepatic cells (US 13/032,521, WO 2004/112824, Simon et al., FASEB J. 2003 Aug; 17(11): 1441-50).

It has also been shown that a 15-amino acid peptide, derived from the Reg IIIa family (HIP/PAP), the HIP (Human proIslet Peptide) peptide, has a regenerating activity on pancreatic islets and thus stimulates insulin production (US 2010/0093605).

The HIP/PAP protein according to the invention can comprise an amino acid sequence selected from the group consisting of sequences set forth as SEQ ID NO: 1, SEQ ID NO: 2, SEQ ID NO: 3 and SEQ ID NO: 4.

The amino acid sequence SEQ ID NO: 1 corresponds to the HIP/PAP protein of sequence SEQ ID NO: 4, from which the N-terminal 26-amino acid signal peptide of said protein has been deleted.

In a particular embodiment, the HIP/PAP protein according to the invention comprises, or consists of, the amino acid sequence set forth as SEQ ID NO: 4.

In a particular embodiment, the HIP/PAP protein according to the invention comprises, or consists of, the amino acid sequence set forth as SEQ ID NO: 1.

The amino acid sequence SEQ ID NO: 2 corresponds to the short form of the HIP/PAP protein and, compared with the amino acid sequence SEQ ID NO: 1, has had the 11-amino acid propeptide in the N-terminal position deleted.

In a particular embodiment, the HIP/PAP protein according to the invention comprises, or consists of, the amino acid sequence set forth as SEQ ID NO: 2.

The sequence SEQ ID NO: 3 corresponds to the sequence SEQ ID NO: 1, to which a methionine has been added in the N-terminal position. This sequence may in particular be produced recombinantly in *E.Coli* cells. The 12-amino acid N-terminal propeptide (propeptide of 11 amino acids plus the additional methionine) may be cleaved, in order to obtain the short form of the HIP/PAP protein (SEQ ID NO: 2).

In a particular embodiment, the HIP/PAP protein according to the invention comprises, or consists of, the amino acid sequence set forth as SEQ ID NO: 3.

According to the invention, the short or long forms of the HIP/PAP protein or of the derivatives thereof may be used indifferently.

A derivative of the HIP/PAP protein according to the invention designates a biologically active derived form of a HIP/PAP protein of any one of sequences SEQ ID No. 1 to 4. The terms *"biologically active"* mean that the derivative of the HIP/PAP protein have the same biological activity as the HIP/PAP protein of any one of sequences SEQ ID No. 1 to 4.

*A derivative of the HIP*/*PAP protein according to the invention comprises, or consists in, an amino acid sequence having a sequence identity of at least 80% with the amino acid sequence selected from the group consisting of sequences SEQ ID NO: 1, SEQ ID NO: 2, SEQ ID NO: 3 and SEQ ID NO: 4 and a biological activity of the same nature as the amino acid sequence selected from the group consisting of sequences SEQ ID NO: 1, SEQ ID NO: 2, SEQ ID NO: 3 and SEQ ID NO: 4.*

A biological activity of the same nature as the HIP/PAP protein according to the present invention is as described previously, i.e. the capacity to treat and/or prevent a disorder characterized by a CXCL5 level higher in a biological sample of the said individual than in a corresponding biological sample of a healthy individual, and in particular of a disorder selected from the group consisting of T helper 17 cell-mediated autoimmune disorders; osteoporosis; osteopetrosis; arthritis; retinopathies and Paget's disease, more particularly selected from the group consisting of multiple sclerosis; rheumatoid arthritis; psoriasis; osteoporosis; osteopetrosis; arthritis; retinopathies and Paget's disease.

As described herein, an amino acid sequence having at least 80% amino acid identity with a reference amino acid sequence encompasses amino acid sequences having at least 81%, 82%, 83%, 84%, 85%, 86%, 87%, 88%, 89%, 90%, 91%, 92%, 93%, 94%, 95%, 96%, 97%, 98% and 99% amino acid identity with the said reference amino acid sequence, and also a biological activity of the same nature as the said reference amino acid sequence.

In certain embodiments, the HIP/PAP protein, or a derivative thereof, may be associated or combined by noncovalent bonds with non-HIP/PAP portions. For example, the HIP/PAP protein or a derivative thereof could be associated with a liposome particle. Depending on the type of liposome, or on the production process, the HIP/PAP protein, or the derivative thereof, may be associated at the surface of the liposome or encapsulated inside said liposome.

The HIP/PAP protein, or a derivative thereof, may also be associated with non-HIP/PAP portions by covalent bonds. Such non-HIP/PAP portions may be selected from protein or non-protein compounds, for example polyethylene glycol, thus forming a pegylated HIP/PAP derivative.

A derivative of the HIP/PAP protein according to the invention also comprises the derivatives which become biologically active only when they are administered to the patient.

Finally, the derivatives of the HIP/PAP protein also comprise chimeric proteins or fusion proteins. Such proteins are fused with non-HIP/PAP polypeptides. The latter may be fused with the N- or C-terminal part. Typically, the HIP/PAP protein or a derivative thereof may be fused with the GST sequence at the level of their C-terminal part, in order to facilitate the purification of the recombinant proteins.

In certain embodiments of the invention, the HIP/PAP protein, or a derivative thereof, according to the invention is produced recombinantly in bacterial or animal cells, including insect and mammalian cells, according to techniques known to those skilled in the art.

In other embodiments, the HIP/PAP protein, or a derivative thereof, according to the invention may be isolated from a cell or from a tissue by known purification techniques.

The HIP/PAP protein and the derivatives thereof may also be produced by chemical synthesis.

In the text, the terms "*protein"* cover the HIP/PAP protein in itself, and also the derivatives thereof as described above.

### Compositions

The invention also relates to the implementation of the HIP/PAP protein, or a derivative thereof, as defined previously in a composition comprising a physiologically acceptable medium.

The physiologically acceptable medium, previously defined in the present text, may be chosen, according to the pharmaceutical form and the mode of administration desired, from the usual excipients which are known to those skilled in the art (see Remington's Pharmaceutical Sciences, 16th edition, Osol, A ed., 1980).

By way of example, compositions according to the invention may comprise, according to the therapeutic indications and the HIP/PAP protein or the derivative thereof:
a) the HIP/PAP protein or a derivative thereof; and
b) a buffer capable of maintaining the pH in a maximum stability range, preferentially from 1 to 9, more particularly from 4 to 8 and even more particularly from 6 to 7.5; and/or
c) a detergent or a surfactant which stabilizes the protein or the polypeptide against the aggregation induced by stirring; and/or
d) an isotonic; and/or
e) a preservative, chosen for example from the group consisting of phenols, benzyl alcohols, benzothelium halides, and chlorides; and/or
f) water.

If the detergent or the surfactant used is nonionic, it may be chosen from polysorbates, PLURONIC TM, polyethylene glycol (PEG) or poloxamers.

An isotonic will make it possible to maintain the isotonicity of the composition and will typically include polyalcohols such as glycerol, erythritol, arabitol, xylitol, sorbitol or mannitol, used alone or in combination. Alternatively, sodium chloride and/or any other inorganic salt may be used as isotonic.

The buffer may be, for example, acetate, citrate, succinate, a phosphate buffer or any other inorganic buffer, depending on the desired pH.

The preservatives of phenol, benzyl alcohol, benzothelium halide and chloride type are known antimicrobial agents. Typical preservatives comprise octadecyldimethylbenzylammonium chloride, hexamethonium chloride, benzalkonium chloride, phenol, butyl or benzyl alcohols, alkyl parabens, such as methyl or propyl paraben, catechol, resorcinol, cyclohexanol, 3-pentanol and m-cresol.

Additional excipients may also comprise antioxidants, such as ascorbic acid and methionine, chelating agents, such as EDTA, sugars, such as sucrose, mannitol, trehalose or sorbitol, etc.

The HIP/PAP protein, or the derivative thereof, according to the invention may be in the form of a pharmaceutically acceptable salt. This is intended to mean salts prepared from pharmaceutically acceptable non-toxic acids or pharmaceutically acceptable non-toxic bases, including organic and inorganic salts and acids. By way of example, mention may be made of alkali metal salts (sodium and potassium salts), alkaline-earth metal salts (calcium and magnesium salts), ammonium salts, salts of organic bases (pyridine or triethylamine salts), salts of inorganic acids (hydrochloride, sulfate, nitrate) and salts of organic acids (acetate, oxalate, p-toluenesulfonate).

A composition implemented according to the invention may be for oral, sublingual, subcutaneous, intramuscular, intravenous, topical, local, intratracheal, intranasal or rectal administration, and in particular for oral, subcutaneous, intravenous, topical or local administration.

According to one preferred embodiment, the HIP/PAP protein is administered in an effective amount, i.e. the amount required to obtain the expected effects of the invention. Such an amount of HIP/PAP protein will be determined, generally empirically, according to the subject to be treated and to his pathological condition. The effective amount will also depend on the mode of administration envisioned. The adjustments required to determine the effective amount for obtaining the maximum therapeutic effect correspond to techniques that are routine for the clinician.

An effective amount of the HIP/PAP protein or of a derivative thereof may for exemple be between 0.1 µg/day/kg of body weight and 100 mg/day/kg of body weight of the individual to which it must be administered. Although in certain embodiments an effective amount of the HIP/PAP protein, or derivative thereof, may reach more than 10 mg/kg, an effective amount of the HIP/PAP protein, or derivative thereof, according to the present invention is generally less than 5 mg/kg of body weight, which includes amounts less than 4.5 mg/kg, 4 mg/kg, 3.5 mg/kg, 3 mg/kg, 2.5 mg/kg or 2000 µg/kg. More particularly, an effective amount of the HIP/PAP protein, or derivative thereof, according to the present invention comprises amounts of at least 1 µg/kg, 2 µg/kg, 3 µg/kg, 4 µg/kg, 5 µg/kg, 6 µg/kg, 7 µg/kg, 8 µg/kg, 9 µg/kg, 10 µg/kg, 15 µg/kg, 20 µg/kg, 25 µg/kg, 30 µg/kg, 40 µg/kg, 50 µg/kg, 60 µg/kg, 70 µg/kg, 80 µg/kg, 90 µg/kg, 100 µg/kg, 150 µg/kg, 200 µg/kg, 250 µg/kg, 300 µg/kg, 350 µg/kg, 400 µg/kg, 450 µg/kg, 500 µg/kg, 600 µg/kg, 700 µg/kg, 800 µg/kg, 900 µg/kg, 1 mg/kg, 2 mg/kg, 3 mg/kg, 4 mg/kg, 5 mg/kg or more with respect to body weight of the individual to which it is or must be administered.

According to particular embodiments, the HIP/PAP protein, or derivative thereof, is administered according to a dosage of between 10 and 5000 µg/kg, preferentially between 100 and 2000 µg/kg with respect to body weight.

In the compositions of the present invention for oral, sublingual, subcutaneous, intramuscular, intravenous, topical, local, intratracheal or intranasal or rectal administration, the active ingredient (the HIP/PAP protein or a derivative thereof) may be administered in unit administration form as a mixture with pharmaceutical excipients.

When the composition is for oral administration, said composition may be chosen from the group consisting of a food product, a beverage, a pharmaceutical product, a nutraceutical, a food additive, a food supplement and a milk product.

The preferred modes of administration are the oral, subcutaneous, intravenous, topical or local routes, and more particularly the subcutaneous, intravenous, topical or local routes.

The administration of a compound or composition of the invention may for example be performed through the use of a sheath, a patch, a pad, a compress, a bandage, a tape, a gauzebased dressing, a woven or non-woven sponge or a syringe.

The HIP/PAP protein, or a derivative thereof, may be sterilized prior to the *in vivo* administration. The sterilization may be obtained by filtration on sterile filtration membranes, before or after the lyophilization or the reconstitution. The systemically administered HIP/PAP protein, or derivative thereof, may advantageously be lyophilized or stored in solution. In lyophilized form, the HIP/PAP protein, or derivative thereof, may be generally formulated in combination with excipients which enable reconstitution with an appropriate diluent, at the time of use.

The HIP/PAP protein, or a derivative thereof, may be administered daily in one intake or in a fractionated manner (for example from 2 to 3 times per day) until the desired therapeutic effect is obtained. It may also be administered chronically.

The HIP/PAP protein, or a derivative thereof, may also be administered in the form of a course, for example courses ranging from 15 days to 3 months, optionally repeated from 1 to 6 times at determined doses and time intervals.

The HIP/PAP protein, or a derivative thereof, according to the invention may also be combined in the context of a polytherapy with other compounds for treating and/or preventing a disorder characterized by a CXCL5 level higher in a biological sample of said individual than in a corresponding biological sample of a healthy individual, and in particular of a disorder selected from the group consisting of T helper 17 cell-mediated autoimmune disorders; osteoporosis; osteopetrosis; arthritis; retinopathies and Paget's disease, more particularly selected from the group consisting of multiple sclerosis; rheumatoid arthritis; psoriasis; osteoporosis; osteopetrosis; arthritis; retinopathies and Paget's disease.

Accordingly, a HIP/PAP protein, a derivative thereof, or a composition comprising it according to the invention, may be administered to the previously defined individual in combination with at least one agent known for being useful in the prevention and/or treatment of a disorder characterized by a CXCL5 level higher in a biological sample of the said individual than in a corresponding biological sample of a healthy individual, and in particular of a disorder selected from the group consisting of T helper 17 cell-mediated autoimmune disorders; osteoporosis; osteopetrosis; arthritis; retinopathies and Paget's disease, more particularly selected from the group consisting of multiple sclerosis; rheumatoid arthritis; psoriasis; osteoporosis; osteopetrosis; arthritis; retinopathies and Paget's disease.

Such agents are known by the man skilled in the art and can for example be selected from:
for multiple sclerosis: teriflunomide (such as AUBAGIO^{®}), interferon beta-la (such as AVONEX^{®}), interferon beta 1b (such as BETAFERON^{®}), betamethasone (such as CELESTENE^{®}) or glatiramer acetate (such as COPAXONE^{®}); and/or
for rheumatoid arthritis: Disease Modifying Anti Rheumatic Drugs (DMARD), non-steroidal antiinflammatory drugs (NSAIDS), corticosteroids, analgesics, adalimumab (such as AMGEVITA^{®}, HULIO^{®} or HUMIRA^{®}) or etanercept (such as BENEPALI^{®} or ERELZI^{®}); and/or
for psoriasis: leflunomide (such as ARAVA^{®}), sulindac (such as ARTHROCINE^{®}); or betamethasone valerate (such as BETESIL^{®}); and/or
for osteoporosis: Zoledronic acid monohydrate (such as ACLASTA^{®}), calcium carbonate (FIXICAL^{®}), teriparatide (such as TERROSA^{®}), monohydrate monosodium alendronic acid (ALENDRONATE TEVA^{®}) or risedronate (such as ACTONEL^{®}); and/or
for osteopetrosis: recombinant interferon gamma-lb (IMUKIN^{®}); and/or
for arthritis: diclofenac epolamine (such as FLECTOR^{®}), diclofenac sodium (such as VOLTARENE^{®}), methotrexate (such as FOLINORAL^{®}), hydroxychloroquine sulfate (such as PLAQUENIL^{®}), sulfasalazine (such as SALAZOPYRINE^{®}) or leflunomide (such as ARAVA^{®}); and/or
for retinopathies: etamsylate (such as DICYNONE^{®}); and/or
for Paget's disease: risedronate sodium (such as ACTONEL^{®}).

By administered in combination with, it means that the HIP/PAP protein, a derivative thereof, or a composition comprising it according to the invention can be administered simultaneously or sequentially compared to the other compound(s). When they are in separate compositions, the composition comprising the HIP/PAP protein or a derivative thereof according to the invention and the composition comprising the at least one other compound can be administered through the same route or through different routes.

The HIP/PAP protein, or a derivative thereof, according to the invention and the at least one other compound can be administered in the same composition or in separate compositions.

By simultaneously, it is understood that the compositions can be administered at the same moment or up to the same day or couple of days.

By sequentially, it is understood that the compositions can be administered with at least several days, for example at least two days of difference.

### Implementation of the HIP/PAP protein and/or of a derivative thereof

As previously mentioned, a HIP/PAP protein, of a derivative thereof, as well as a composition comprising it, is for use in the treatment and/or prevention, in an individual in need thereof, of a disorder characterized by a CXCL5 level higher in a biological sample of the said individual than in a corresponding biological sample of a healthy individual.

Methods for determining the level of CXCL5 are well-known by the man skilled in the art. For illustrative purposes, can be mentioned Chavey et al. (Aging (Albany NY) 2009 Jul 2; 1(7):674-7) wherein CXCL5 concentration in cellular extracts, conditioned medium and serum was determined by ELISA with Duoset kit (DY254 and DY443, R&D Systems).

As previously indicated, the biological samples of the individual with a disorder and of the healthy individual are liquid samples or tissue samples, and are in particular liquid samples, more particularly selected from the group consisting of whole blood, plasma, serum, urine and saliva samples, in particular serum or urine samples and more particularly serum samples.

A tissue sample of the invention is in particular selected from the group consisting of a brain tissue, in particular a tissue from the olfactory bulb, amygdala, basal ganglia, thalamus, midbrain, pons, medulla oblongata, hippocampal formation, spinal cord, white matter, cerebral cortex, cerebellum, choroid plexus or hypothalamus; an endocrine tissue, such as a tissue from thyroid gland, parathyroid gland, adrenal grland or pituitary gland; a respiratory system tissue, such as a tissue from the nasopharynx, bronchus or lung; a digestive tract tissue, such as a tissue from oral mucosa, salivary gland, esophagus or tongue; a gastrointestinal tissue, in particular a tissue from the stomach, colon, duodenum, rectum or small intestine; a liver tissue; a pancreas tissue; a kidney tissue; a bladder tissue; a urinary tract tissue; a tissue from testis, epididymis, prostate or seminal vesicle; a tissue from the vagina, uterus, cervix, breast, endometrium, fallopian tube, ovary or placenta tissue; a muscle tissue, such as a tissue from heart muscle, skeletal muscle or smooth muscle; a skin tissue; and a bone marrow or lymphoid tissue, such as a tissue from the bone marrow, the appendix, lymph node, spleen, thymus or tonsil.

The nature and origin of the samples is appropriately selected by the man skilled in the art according to the nature of the disorder. Indeed, the man skilled in the art knows, as for example illustrated herein, in which liquid sample and/or tissue sample of the individual with a disorder the CXCL5 concentration is elevated, i.e. is higher than a corresponding biological sample in a healthy individual.

A disorder to be treated or prevented according to the invention may in particular be selected from the group consisting of T helper 17 cell-mediated autoimmune disorders; osteoporosis; osteopetrosis; arthritis; retinopathies and Paget's disease, and in particular selected from the group consisting of multiple sclerosis; rheumatoid arthritis; psoriasis; osteoporosis; osteopetrosis; arthritis; retinopathies and Paget's disease.

Indeed, Rumble et al. (J Exp Med. 2015 Jan 12;212(1):23-35) describes that elevated plasma levels of CXCL5 are correlated with measures of multiple sclerosis (MS) lesion burden, clinical disability, acute lesion formation and relapsing MS patients coincident with acute lesion development. They in particular found that expression of CXCL5 was elevated in active patients concomitant with the presence of acute inflammatory lesions on MRI scan when compared with inactive patients with no inflammatory lesions.

Moreover, E. Smith et al. (Arthritis Rheum. 2008 Jul;58(7): 1968-73) describes that CXCL5 plays an important role in rheumatoid arthritis (RA) because it is abundant in RA tissue, and its neutralization moderates joint damage in animal models.

Guilloteau et al. (J Immunol 2010; 184:5263-5270) disclosed that the expression of CXCL5 encoding genes are strongly increased in psoriatic skin compared with normal skin.

Kawai et al. (J Intern Med. 2012 Oct;272(4):317-29) describes that there is an intimate association between bone cells and adipocytes. Adipose tissue has important regulatory circuits that modulate skeletal remodeling including the direct effect of cytokines on bone cells due to infiltrating macrophages (Jay J Cao (J Orthop Surg Res. 2011; 6: 30)), which are known for highly expressing CXCL5 (Dabin Lee et al. J Lipid Res. 2021; 62: 100117), and illustrate the link between an increase in CXCL5 and osteoporosis and osteopetrosis.

Laura J. Brylka and Thorsten Schinke (Front Immunol. 2019; 10: 2182.) described the correlation between alteration of bone remodeling and an increased expression of CXCL5, induced by IL-17 in osteoblasts. Paget's disease patients in particular displayed a 5-fold increase of CXCL5 serum levels.
C. Chavey and L. Fajas (Aging (Albany NY). 2009 Jul 2;1(7):674-7) describes that CXCL5 is implicated in the development of retinopathies considering the increased levels of this chemokine found in retinopathy patients.

Moreover, Pickens et al. (Angiogenesis. 2011 Dec;14(4):443-55) demonstrated that IL-17-mediated disease activity was not affected by anti-CXCL1 treatment alone but that in contrast, mice receiving anti-CXCL5 demonstrated significantly reduced clinical signs of arthritis, compared to the mice treated with IgG control. They indeed found that arthritis severity and vascularization were significantly reduced in the anti-CXCL5 treatment group.

A disorder to be treated or prevented according to the invention may in particular be selected from the group consisting of T helper 17 cell-mediated autoimmune disorders; arthritis; retinopathies; and Paget's disease, and in particular selected from the group consisting of multiple sclerosis; rheumatoid arthritis; psoriasis; arthritis; retinopathies and Paget's disease, and may more particularly be selected from the group consisting of multiple sclerosis; rheumatoid arthritis; psoriasis; arthritis and Paget's disease.

An individual to which the HIP/PAP protein, a derivative thereof, or a composition comprising it according to the invention is administered can in particular be a mammal and in particular a human being.

The HIP/PAP protein, a derivative thereof, or a composition according to the invention can be administered in combination with standards of medical care for the considered disorders mentioned above.

The present invention also relates to a method for treating and/or preventing, in an individual in need thereof, a disorder characterized by a CXCL5 level higher in a biological sample of the said individual than in a corresponding biological sample of a healthy individual, in particular a disorder selected from the group consisting of T helper 17 cell-mediated autoimmune disorders; osteoporosis; osteopetrosis; arthritis; retinopathies and Paget's disease, and more particularly selected from the group consisting of multiple sclerosis; rheumatoid arthritis; psoriasis; osteoporosis; osteopetrosis; arthritis and Paget's disease, comprising administering to the said individual a HIP/PAP protein, a derivative thereof, or a composition comprising it.

The present invention moreover relates to the use of a HIP/PAP protein, a derivative thereof, or a composition comprising it, for treating and/or preventing, in an individual in need thereof, a disorder characterized by a CXCL5 level higher in a biological sample of the said individual than in a corresponding biological sample of a healthy individual.

### Particular methods of the invention

As previously indicated, the present invention also relates to a method, in particular *in vitro* or *ex vivo,* for measuring a response to a HIP/PAP protein, or a derivative thereof, in an individual having a disorder characterized by a CXCL5 level higher in a biological sample of said individual than in a corresponding biological sample of a healthy individual.

As previously indicated, the biological samples of the individual with a disorder and of the healthy individual are liquid samples or tissue samples, and are in particular liquid samples, more particularly selected from the group consisting of whole blood, plasma, serum, urine and saliva samples, in particular serum or urine samples and more particularly serum samples.

A tissue sample of the invention is in particular selected from the group consisting of a brain tissue, in particular a tissue from the olfactory bulb, amygdala, basal ganglia, thalamus, midbrain, pons, medulla oblongata, hippocampal formation, spinal cord, white matter, cerebral cortex, cerebellum, choroid plexus or hypothalamus; an endocrine tissue, such as a tissue from thyroid gland, parathyroid gland, adrenal grland or pituitary gland; a respiratory system tissue, such as a tissue from the nasopharynx, bronchus or lung; a digestive tract tissue, such as a tissue from oral mucosa, salivary gland, esophagus or tongue; a gastrointestinal tissue, in particular a tissue from the stomach, colon, duodenum, rectum or small intestine; a liver tissue; a pancreas tissue; a kidney tissue; a bladder tissue; a urinary tract tissue; a tissue from testis, epididymis, prostate or seminal vesicle; a tissue from the vagina, uterus, cervix, breast, endometrium, fallopian tube, ovary or placenta tissue; a muscle tissue, such as a tissue from heart muscle, skeletal muscle or smooth muscle; a skin tissue; and a bone marrow or lymphoid tissue, such as a tissue from the bone marrow, the appendix, lymph node, spleen, thymus or tonsil.

The nature and origin of the samples is appropriately selected by the man skilled in the art according to the nature of the disorder. Indeed, the man skilled in the art knows, as for example illustrated herein, in which liquid sample and/or tissue sample of the individual with a disorder the CXCL5 concentration is elevated, i.e. is higher than a corresponding biological sample in a healthy individual.

This method comprises a first step (a) consisting in determining the CXCL5 level in a biological sample of the individual. Methods and tools to perform this measure are well known to the man skilled in the art as indicated previously. For example, CXCL5 levels, and in particular serum CXCL5 levels, can be assayed by enzyme-linked immunosorbent assay (ELISA) using Quantikine Human ENA-78 (R&D Systems, Minneapolis, MN) in accordane with the manufacturer's instructions.

Following step (a), the method of the invention comprises the step (b) of administering to the said individual a HIP/PAP protein, or a derivative thereof.

As mentioned above, the HIP/PAP protein, or a derivative thereof, may be in a composition comprising a physiologically acceptable medium.

Moreover, all the elements detailed above concerning the administration of the HIP/PAP protein, or a derivative thereof, or a composition comprising it, apply here, including administration route, doses and regimens.

Step (b) of the method of the invention may for example last for at least 7 days, in particular at least 2 weeks, more particularly at least 3 weeks.

Following step (b), the method comprises step (c) wherein the CXCL5 level of the individual treated according to step (b) is determined in a biological sample corresponding to the biological sample of step (a), i.e. a biological sample having the same biological nature (liquid sample or tissue sample) and the same biological origin as the biological sample of step (a).

A similar method as the one mentioned for step (a) to perform this step may be applied.

Step (c) is then followed by step (d) consisting in comparing the CXCL5 level determined at step (a) with the CXCL5 level determined at step (c).

As mentioned above, a method for determining this CXCL5 level is known in the prior art.

When the comparition of the two CXCL5 levels of steps (a) and (c) shows a diminution of the CXCL5 level between step (a) and step (c) (i.e. the level of CXCL5 determined at step (a) is superior to the level of CXCL5 determined at step (c)), then a response to the administration of HIP/PAP protein, or a derivative thereof, is observed.

When the comparition of the two CXCL5 levels of steps (a) and (c) shows no significant variation or an increase of the CXCL5 level between step (a) and step (c) (i.e. the level of CXCL5 determined at step (a) is equal or inferior to the level of CXCL5 determined at step (c)), then no response according to the invention to the administration of HIP/PAP protein, or a derivative thereof, is observed.

The present invention also relates to another method, *in vitro* or *ex vivo,* for determining if a disorder in an individual can be treated by the administration, to the said individual, of a HIP/PAP protein, or a derivative thereof.

As mentioned above, the HIP/PAP protein, or a derivative thereof, may be in a composition comprising a physiologically acceptable medium.

The method comprises a first step (a) consisting in determining the CXCL5 level of CXCL5 in a biological sample of the individual. The CXCL5 level in step (a) of the method may in particular be determined in a serum sample of the individual.

Methods and tools to perform this measure are well known to the man skilled in the art as mentioned several times above in the present text.

The method further comprises a step (b) consisting in comparing the CXCL5 level determined at step (a) with the CXCL5 level of a corresponding biological sample of an healthy individual i.e. a biological sample having the same biological nature (liquid sample or tissue sample) and the same biological origin as the sample of step (a). In particular, the sample of step (a) and the sample of step (b) of the method are serum samples.

The CXCL5 level, and in particular the CXCL5 serum level, of an healthy individual can be a reference value already available. It can also be determined using a biological sample of the said individual dating from before he was affected by the disorder.

As previously indicated, the biological samples of the individual with a disorder and of the healthy individual are liquid samples or tissue samples, and are in particular liquid samples, more particularly selected from the group consisting of whole blood, plasma, serum, urine and saliva samples, in particular serum or urine samples and more particularly serum samples.

A tissue sample of the invention is in particular selected from the group consisting of a brain tissue, in particular a tissue from the olfactory bulb, amygdala, basal ganglia, thalamus, midbrain, pons, medulla oblongata, hippocampal formation, spinal cord, white matter, cerebral cortex, cerebellum, choroid plexus or hypothalamus; an endocrine tissue, such as a tissue from thyroid gland, parathyroid gland, adrenal grland or pituitary gland; a respiratory system tissue, such as a tissue from the nasopharynx, bronchus or lung; a digestive tract tissue, such as a tissue from oral mucosa, salivary gland, esophagus or tongue; a gastrointestinal tissue, in particular a tissue from the stomach, colon, duodenum, rectum or small intestine; a liver tissue; a pancreas tissue; a kidney tissue; a bladder tissue; a urinary tract tissue; a tissue from testis, epididymis, prostate or seminal vesicle; a tissue from the vagina, uterus, cervix, breast, endometrium, fallopian tube, ovary or placenta tissue; a muscle tissue, such as a tissue from heart muscle, skeletal muscle or smooth muscle; a skin tissue; and a bone marrow or lymphoid tissue, such as a tissue from the bone marrow, the appendix, lymph node, spleen, thymus or tonsil.

The nature and origin of the samples is appropriately selected by the man skilled in the art according to the nature of the disorder. Indeed, the man skilled in the art knows, as for example illustrated herein, in which liquid sample and/or tissue sample of the individual with a disorder the CXCL5 concentration is elevated, i.e. is higher than a corresponding biological sample in a healthy individual.

The disorder of the individual in the method may be selected from the group consisting of:
(i) traumatic brain injury (TBI); foetal, neonate, child or adult traumatic brain injury, in particular caused by a brain hypoxia; cerebellar disease or disorder, such as cerebellar ataxias; Alzheimer's disease; pathological states that precede the occurrence of the Alzheimer's disease *per se,* which includes mind cognitive impairment; stroke, in particular ischemic stroke and hemorrhagic stroke. HIP/PAP or its derivatives have indeed already been described as being able to prevent or treat these disorders (see for example EP2440225A1).
(ii) cancers, and in particular lung cancer, including small-cell lung cancer, non-small cell lung cancer, adenocarcinoma of the lung and squamous carcinoma of the lung; cancer of the peritoneum; hepatocellular cancer; gastric or stomach cancer, including gastrointestinal cancer; pancreatic cancer; glioblastoma; cervical cancer; ovarian cancer; liver cancer; bladder cancer; cancer of the urinary tract; breast cancer; colon cancer; rectal cancer; colorectal cancer; small intestine cancer; endometrial or uterine carcinoma; salivary gland carcinoma; kidney or renal cancer; prostate cancer; vulval cancer; thyroid cancer; hepatic carcinoma; anal carcinoma; penile carcinoma; brain tumor; head and neck cancers and melanoma. HIP/PAP or its derivatives have indeed already been described as being able to prevent or treat these cancers (see for example EP2874645A1).
(iii) insulin resistance; dyslipidemia, in particular hypercholesterolemia; atherosclerosis, in particular a coronary artery disease and a cerebrovacular disease or lower extremity arterial disease. HIP/PAP or its derivatives have indeed already been described as being able to prevent or treat these cancers (see for example EP2983784A1).
(iv) visceral excess weight; obesity; metabolic syndrome; polycystic ovary syndrome; an eating disorder; hepatitis C; hepatic steatosis; sarcopenia; hyperandrogenism; hypercatabolism and undernourishment. HIP/PAP or its derivatives have indeed already been described as being able to prevent or treat these cancers (see for example EP2983784A1).
(v) microbiota-related diseases and disorders, and in particular disease or disorder selected from the group consisting of inflammatory bowel disease (IBD); colitis; gastrointestinal infections; irritable bowel syndrome and other gastrointestinal functional diseases; allergic diseases; cystic fibrosis; atopic dermatitis; neurological diseases, such as autism; anxiety; depression and chronic pain. HIP/PAP or its derivatives have indeed already been described as being able to prevent or treat these cancers (see for example EP3651787A1)
(vi) peripheral neuropathies, in particular diabetic peripheral neuropathies, as HIP/PAP or its derivatives have indeed already been described as being able to prevent or treat these disorders.
(vii) T helper 17 cell-mediated autoimmune disorders, in particular multiple sclerosis; rheumatoid arthritis; psoriasis; osteoporosis; osteopetrosis; arthritis; retinopathies and Paget's disease.

When the CXCL5 level of step (a) is determined as being superior to the CXCL5 level of reference (i.e. the CXCL5 level of a corresponding biological sample of a healthy individual), then the disorder can be treated by the administration of HIP/PAP protein, or a derivative thereof, to the individual having said disorder.

When the CXCL5 level of step (a) is determined as being inferior or equal (i.e. not significantly different) to the CXCL5 level of reference (i.e. the CXCL5 level of a corresponding biological sample of a healthy individual), then this method does not allow do conclude that the disorder can be treated by the administration of HIP/PAP protein, or a derivative thereof, to the individual having said disorder.

The invention is described below in greater detail by means of the following examples which are given solely by way of illustration.

All the references to percentages are percentages by weight unless otherwise indicated.

### EXAMPLES

Effect of a 28-day subcutaneous treatment with HIP/PAP on systemic inflammation in mouse model

### Mouse model

### a. Animals

4-weeks old C57B1/6 male mice were housed and fed by Janvier Labs for 7 weeks prior to be sent to the University of Paris' animal facility (Paris, France). Experiments were performed under the institutional and European Union guidelines for laboratory animal care. The animals were housed in a barriered rodent unit, under controlled standard laboratory conditions.

### b. Diet

4 weeks old mice were fed with HFD 260 (Safe). Food is changed 3 times a week. The food was provided ad libitum and placed in the plastic feeder on top of the cage. Water was also provided ad libitum from water bottles equipped with rubber stoppers and sipper tubes. Water bottles were changed once a week.

The high-fat diet-fed (HFD) mouse model is a model of obesity and glucose homeostasis dysregulation (prediabetes) where mice are fed with a diet highly enriched in lipids and in saccharose (see Winzell, M. S. & Ahrén, B. Diabetes 53 Suppl 3, S215-219 (2004))

As a consequence, HFD mouse model displays insulin-resistance with hyperinsulinemia, obesity, hyperglycemia and hyperlipidemia (see Migrenne, S. et al. Am. J. Physiol. Regul. Integr. Comp. Physiol. 296, R929-935 (2009)).

### Treatment

Alzet^{®} pumps 2004 (Charles River Lab France, 1'Arbresle, France) were filled either with HIP/PAP protein of sequence set forth as SEQ ID NO: 3 (N=8)(6.5mg/mL) or with a buffer consisting of 20mM Sodium Phosphate, 130 mM NaCl, pH6.5 (N=7). With a capacity of 200µL and a flow rate of 0.25µL/hr, pumps filled with 6.5 mg/mL of the tested HIP/PAP protein deliver continuously 39 µg/d for 28 days.

C57B1/6 HFD mice were anesthetized on day 1. Their skin was shaved and washed over the implantation site. A mid-scapular incision was made, and a hemostat was inserted into the incision. By opening and closing the jaws of the hemostat, the subcutaneous tissue was spread to create a pocket for the pump. The pump was then inserted into the subcutaneous pocket with the delivery portal (flow moderator) first. Finally, the wound was closed with sutures.

At the end of the 28 days of treatment, tissues were collected and stored in the appropriate conditions for further analysis.

### Dosage of circulating cytokines

Blood was sampled to dose circulating cytokines using bead-based multiplex immune-assays. The following 32 analytes classically involved in inflammation or immune system were dosed: G-CSF, GM-CSF, IFN-γ, IL-1a, IL-1β, IL-2, IL-3, IL-4, IL-5, IL-6, IL-7, IL-9, IL-10, IL-12 (p40), IL-12 (p70), IL-13, IL-15, IL-17, IP-10, KC, LIF, LIX (mouse analog of the human CXCL chemokine), MCP-1, M-CSF, MIG, MIP-1α, MIP-1β, MIP-2, RANTES, TNF-α, VEGF, Eotaxin/CCL11 (MCYTOMAG-70K, Millipore). The assay was analyzed using Luminex technology and the experiment was performed by the platform of the center of research of "Les Cordeliers" (http://www.crc.jussieu.fr/plates_formes.html).

### Data analysis

For each experiment, data from treated animals are compared to control animals (placebo). For each statistical test, the normality of the distributions was assessed prior to choose the appropriate tests and post-hoc tests

### Results

4 weeks old C57B1/6 male mice were fed for 8 weeks with a high fat diet (HFD) composed of 60% of fat energy. From week 8 of diet, mice were treated either with placebo or with a HIP/PAP protein by osmotic pumps placed under the skin. Prior starting the treatment and at the end of the treatment, blood was sampled to dose circulating cytokines. A panel of 32 analytes were dosed in multiplex using the Luminex technology. The methodology used to analyze raw data of fluorescence is the following:

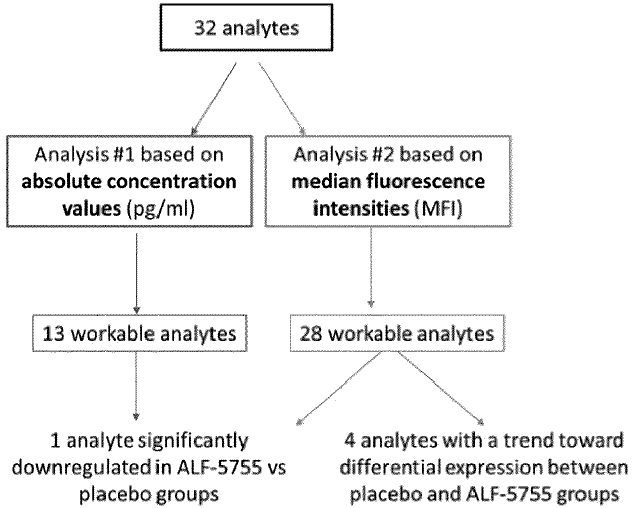

Data were first analyzed using the typical absolute concentration values derived from standard curve of each analyte. Such analysis gave 13 biomarkers out of 32 with workable values, the others being under the minimum value threshold. To decrease the percentage of out-of-range values, we performed a second analysis based on the median fluorescence intensities (MFI) as described in (Breen, E. J., Polaskova, V. & Khan, A. Cytokine 71, 188-198 (2015)). This second analysis allowed to work with 28 analytes out of 32.

Figure 1 shows analytes from analysis #1 (Figure 1A) and #2 (Figure 1B) with significant differential expression between placebo and HIP/PAP protein treated mice concerning the analyte LIX (mouse analog of the human CXCL5 chemokine). LIX is significantly downregulated in HIP/PAP animals compared to placebo as demonstrated by both analysis #1 and #2 (Mann-Whitney, ^{∗∗}p<0.0022).

Among all the 32 studied pro-inflammatory analytes, LIX, or CXCL5, is the only one significantly modulated between placebo and HIP/PAP protein treated mice. For the other studied analytes, only at best a tendency was observed, or even no difference.

### Conclusion

The inventors were able to identify and demonstrate that the analytes LIX/CXCL5 was significantly downregulated in HIP/PAP-treated animals compared to placebo and was associated with an improvement of the animals condition, i.e. an improvement of the insulin resistance of the animals.

This analyte is of particular interest since it has been shown to be upregulated in a significant number of disorders detailed above in the present text.

The present results show that the treatment with HIP/PAP or a derivative thereof is of high interest in the prevention or treatment of disorder characterized by a CXCL5 level higher in the individual affected by said disorder than in a healthy individual, i.e. an individual not affected by said disorder, and in particular not affected by any disorder characterized by a CXCL5 level higher in a biological sample of the individual compared to a corresponding biological sample of a healthy patient.

### SEQUENCE LISTING

**SEQ ID NO: 1** is the amino acid sequence of the HIP/PAP protein from which the N-terminal 26-amino acid signal peptide has been deleted

EEPQRELPSARIRCPKGSKAYGSHCYALFLSPKSWTDADLACQKRPSGNLVSVLSGAE
GSFVSSLVKSIGNSYSYVWIGLHDPTQGTEPNGEGWEWSSSDVMNYFAWERNPSTIS
SPGHCASLSRSTAFLRWKDYNCNVRLPYVCKFTD

**SEQ ID NO: 2** is the amino acid sequence of the HIP/PAP protein from which the N-terminal 26-amino acid signal peptide and the 11-amino acid propeptide in the N-terminal position have been deleted

IRCPKGSKAYGSHCYALFLSPKSWTDADLACQKRPSGNLVSVLSGAEGSFVSSLVKSI
GNSYSYVWIGLHDPTQGTEPNGEGWEWSSSDVMNYFAWERNPSTISSPGHCASLSRS
TAFLRWKDYNCNVRLPYVCKFTD

**SEQ ID NO: 3** is the amino acid sequence of the HIP/PAP protein from which the N-terminal 26-amino acid signal peptide has been deleted and a methionine added in N-terminal position

MEEPQRELPSARIRCPKGSKAYGSHCYALFLSPKSWTDADLACQKRPSGNLVSVLSG
AEGSFVSSLVKSIGNSYSYVWIGLHDPTQGTEPNGEGWEWSSSDVMNYFAWERNPST
ISSPGHCASLSRSTAFLRWKDYNCNVRLPYVCKFTD

**SEQ ID NO: 4** is the amino acid sequence of the full HIP/PAP protein

MLPPMALPSVSWMLLSCLMLLSQVQGEEPQRELPSARIRCPKGSKAYGSHCYALFLS
PKSWTDADLACQKRPSGNLVSVLSGAEGSFVSSLVKSIGNSYSYVWIGLHDPTQGTE
PNGEGWEWSSSDVMNYFAWERNPSTISSPGHCASLSRSTAFLRWKDYNCNVRLPYV
CKFTD

Amino acid sequences are disclosed in the present specification that serve as references. The same sequences are also presented in a sequence listing formatted according to standard requirements for the purpose of patent matters. In case of any sequence discrepancy with the standard sequence listing, the sequences described in the present specification shall be the reference.

## Claims

1. A HIP/PAP protein, or a derivative thereof, for its use in treating and/or preventing, in an individual in need thereof, a disorder **characterized by** a CXCL5 level higher in a biological sample of said individual than in a corresponding biological sample of a healthy individual, the biological sample being in particular selected from the group consisting of a liquid sample and a tissue sample, more particularly a liquid sample, in particular selected from the group consisting of whole blood sample, plasma sample, serum sample, urine sample and saliva sample, and in particular being a serum sample.

2. The HIP/PAP protein, or a derivative thereof, for use according to claim 1, wherein the disorder is selected from the group consisting of T helper 17 cell-mediated autoimmune disorders; osteoporosis; osteopetrosis; arthritis; retinopathies and Paget's disease.

3. The HIP/PAP protein, or a derivative thereof, for use according to claim 2, wherein the T helper 17 cell-mediated autoimmune disorders are selected from the group consisting of multiple sclerosis, rheumatoid arthritis and psoriasis.

4. The HIP/PAP protein, or a derivative thereof, for use according to any one of claims 1 to 3, **characterized in that** the individual is a mammal, in particular a human being.

5. The HIP/PAP protein, or a derivative thereof, for use according to any one of claims 1 to 4, **characterized in that** the HIP/PAP protein comprises an amino acid sequence selected from the group consisting of sequences set forth as SEQ ID NO: 1, SEQ ID NO: 2, SEQ ID NO: 3 and SEQ ID NO: 4, in particular comprises, and more particularly consists in, the sequence set forth as SEQ ID NO: 3.

6. The HIP/PAP protein, or a derivative thereof, for use according to any one of claims 1 to 5, wherein said derivative comprises an amino acid sequence having a sequence identity of at least 80% with the amino acid sequence selected from the group consisting of sequences SEQ ID NO: 1, SEQ ID NO: 2, SEQ ID NO: 3 and SEQ ID NO: 4 and a biological activity of the same nature as the amino acid sequence selected from the group consisting of sequences SEQ ID NO: 1, SEQ ID NO: 2, SEQ ID NO: 3 and SEQ ID NO: 4, and in particular comprises, and more particularly consists in, an amino acid sequence having a sequence identity of at least 80% with the amino acid sequence set forth as SEQ ID NO: 3 and a biological activity of the same nature as the amino acid sequence set forth as SEQ ID NO: 3.

7. The HIP/PAP protein, or a derivative thereof, for use according to any one of claims 1 to 6, **characterized in that** the HIP/PAP protein, or a derivative thereof, is in a composition comprising a physiologically acceptable medium.

8. The HIP/PAP protein, or a derivative thereof, for use according to claim 7, wherein the composition is for oral, sublingual, subcutaneous, intramuscular, intravenous, topical, local, intratracheal, intranasal or rectal administration.

9. The HIP/PAP protein, or a derivative thereof, for use according to claim 7 or 8, wherein the composition is for oral, subcutaneous, intravenous, topical or local administration.

10. The HIP/PAP protein, or a derivative thereof, for use according to any one of claims 1 to 9, wherein the HIP/PAP protein, or the derivative thereof, is administered to the individual in combination with at least one agent known for being useful in the prevention and/or treatment of the disorder of the individual, and in particular in combination with at least one agent known for being useful in the prevention and/or treatment of a T helper 17 cell-mediated autoimmune disorder, in particular selected from the group consisting of multiple sclerosis, rheumatoid arthritis, or psoriasis; osteoporosis; osteopetrosis; arthritis; retinopathies and Paget's disease.

11. A method for measuring a response to a HIP/PAP protein, or a derivative thereof, in an individual having a disorder **characterized by** a CXCL5 level higher in a biological sample of said individual than in a corresponding biological sample of a healthy individual comprising:
(a) determining the CXCL5 level in the biological sample of the individual, in particular in a serum sample;
(b) after step (a), administering to the said individual a HIP/PAP protein, or a derivative thereof;
(c) determining the CXCL5 level, in a biological sample corresponding to the biological sample of step (a), of the individual treated according to step (b) with a HIP/PAP protein, or a derivative thereof; and
(d) comparing the CXCL5 level determined at step (a) with the CXCL5 level determined at step (c).

12. The method according to claim 11, wherein the HIP/PAP protein, or a derivative thereof, is in a composition comprising a physiologically acceptable medium.

13. The method according to claim 11 or 12, wherein step (b) lasts for at least 7 days, in particular at least 2 weeks, more particularly at least 3 weeks.

14. A method for determining if a disorder in an individual can be treated by the administration, to the said individual, of a HIP/PAP protein, or a derivative thereof, comprising:
(a) determining the level of CXCL5 in a biological sample of the individual, in particular in a serum sample; and
(b) comparing the CXCL5 level determined at step (a) with the CXCL5 level of a corresponding biological sample of a healthy individual.

15. The method according to any one of claims 11 to 14, wherein the disorder is selected from the group consisting of:
- traumatic brain injury (TBI); foetal, neonate, child or adult traumatic brain injury, in particular caused by a brain hypoxia; cerebellar disease or disorder, such as cerebellar ataxias; Alzheimer's disease; pathological states that precede the occurrence of the Alzheimer's disease *per se,* which includes mind cognitive impairment; stroke, in particular ischemic stroke and hemorrhagic stroke;
- cancers, and in particular lung cancer, including small-cell lung cancer, non-small cell lung cancer, adenocarcinoma of the lung and squamous carcinoma of the lung; cancer of the peritoneum; hepatocellular cancer; gastric or stomach cancer, including gastrointestinal cancer; pancreatic cancer; glioblastoma; cervical cancer; ovarian cancer; liver cancer; bladder cancer; cancer of the urinary tract; breast cancer; colon cancer; rectal cancer; colorectal cancer; small intestine cancer; endometrial or uterine carcinoma; salivary gland carcinoma; kidney or renal cancer; prostate cancer; vulval cancer; thyroid cancer; hepatic carcinoma; anal carcinoma; penile carcinoma; brain tumor; head and neck cancers; melanoma;
- insulin resistance; dyslipidemia, in particular hypercholesterolemia; atherosclerosis, in particular a coronary artery disease, a cerebrovacular disease or lower extremity arterial disease;
- visceral excess weight; obesity; metabolic syndrome; polycystic ovary syndrome; an eating disorder; hepatitis C; hepatic steatosis; sarcopenia; hyperandrogenism; hypercatabolism; undernourishment;
- microbiota-related diseases and disorders, and in particular disease or disorder selected from the group consisting of inflammatory bowel disease (IBD); colitis; gastrointestinal infections; irritable bowel syndrome and other gastrointestinal functional diseases; allergic diseases; cystic fibrosis; atopic dermatitis; neurological diseases, such as autism; anxiety; depression; chronic pain;
- peripheral neuropathies, in particular diabetic peripheral neuropathies; and
- T helper 17 cell-mediated autoimmune disorders, in particular multiple sclerosis; rheumatoid arthritis; psoriasis; osteoporosis; osteopetrosis; arthritis; retinopathies and Paget's disease.
